# EUROPEAN PATENT SPECIFICATION

(11) **EP 2 218 461 B1**
(45) Date of publication and mention of the grant of the patent: **20.04.2016**
(21) Application number: 10162787.5
(22) Date of filing: 09.12.2004
(51) Int. Cl.: A61K 39/395, A61P 35/00, C07K 16/28, A61K 39/00, A61K 31/00

(54) **CD40 antibody formulation and methods**
CD40-Antikörper-Formulierung und Verfahren
Formulation d'anticorps CD40 et méthodes

(30) Priority: 22.12.2003 US 531639 P
(43) Date of publication of application: 18.08.2010
(62) Divisional of application: 04801378.3
(73) Proprietor: Pfizer Products Inc., Groton, CT 06340 (US)
(72) Inventor: Bedian, Vahe, Groton, CT 06340 (US); Cusmano, John, Daniel, Groton, CT 06340 (US); Gladue, Ronald, Paul, Groton, CT 06340 (US)
(74) Representative: Pfizer

(56) References cited:
- WO-A1-02/088186
- WO-A2-02/30463
- US-B1- 6 267 958
- SHIRE ST ET AL: "Current trends in monoclonal antibody development and manufacturing", SPRINGER, XP002622309,
- ROWE RC, SHESKEY PJ, WELLER PJ: "Handbook of pharmaceutical excipients", 1 January 2003 (2003-01-01), Pharmaceutical press, london * page 5 - page 6 * * page 556 - page 559 * * page 479 - page 483 *

### SEQUENCE LISTING

<110> Gladue et al., Ronald P.
<120> CD40 ANTIBODY FORMULATION AND METHODS
<130> PC32065A
<160> 10
<170> PatentIn version 3.2
<210> 1
   <211> 126
   <212> PRT
   <213> 3.1.1: Human
<400> 1
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Lys Asp Gly Gly Asn Lys Tyr His Ala Asp Ser Val
       50                  55                  60
   Lys Gly Arg Phe Thr Ile Ser Arg Asp Asn Ser Lys Asn Ala Leu Tyr
   65                  70                  75                  80
   Leu Gln Met Asn Ser Leu Arg Val Glu Asp Thr Ala Val Tyr Tyr Cys
                   85                  90                  95
   Val Arg Arg Gly His Gln Leu Val Leu Gly Tyr Tyr Tyr Tyr Asn Gly
               100                 105                 110
   Leu Asp Val Trp Gly Gln Gly Thr Thr Val Thr Val Ser Ser
           115                 120                 125
<210> 2
   <211> 326
   <212> PRT
   <213> 3.1.1: Human
<400> 2
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val Glu Arg Lys Cys Cys Val Glu Cys Pro Pro Cys Pro Ala Pro
               100                 105                 110
   Pro Val Ala Gly Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
       130                 135                 140
   Val Ser His Glu Asp Pro Glu Val Gln Phe Asn Trp Tyr Val Asp Gly
   145                 150                 155                 160
   Val Glu Val His Asn Ala Lys Thr Lys Pro Arg Glu Glu Gln Phe Asn
                   165                 170                 175
   Ser Thr Phe Arg Val Val Ser Val Leu Thr Val Val His Gln Asp Trp
               180                 185                 190
   Leu Asn Gly Lys Glu Tyr Lys Cys Lys Val Ser Asn Lys Gly Leu Pro
           195                 200                 205
   Ala Pro Ile Glu Lys Thr Ile Ser Lys Thr Lys Gly Gln Pro Arg Glu
       210                 215                 220
   Pro Gln Val Tyr Thr Leu Pro Pro Ser Arg Glu Glu Met Thr Lys Asn
   225                 230                 235                 240
   Gln Val Ser Leu Thr Cys Leu Val Lys Gly Phe Tyr Pro Ser Asp Ile
                   245                 250                 255
   Ala Val Glu Trp Glu Ser Asn Gly Gln Pro Glu Asn Asn Tyr Lys Thr
               260                 265                 270
   Thr Pro Pro Met Leu Asp Ser Asp Gly Ser Phe Phe Leu Tyr Ser Lys
           275                 280                 285
   Leu Thr Val Asp Lys Ser Arg Trp Gln Gln Gly Asn Val Phe Ser Cys
       290                 295                 300
   Ser Val Met His Glu Ala Leu His Asn His Tyr Thr Gln Lys Ser Leu
   305                 310                 315                 320
   Ser Leu Ser Pro Gly Lys
   325
<210> 3
   <211> 112
   <212> PRT
   <213> 3.1.1: Human
<400> 3
   Asp Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Tyr Ser
               20                  25                  30
   Asn Gly Tyr Asn Phe Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Leu Glu Ala Glu Asp Val Gly Val Tyr Tyr Cys Met Gln Ala
                   85                  90                  95
   Leu Gln Thr Pro Arg Thr Phe Gly Gln Gly Thr Lys Val Glu Ile Lys
               100                 105                 110
<210> 4
   <211> 107
   <212> PRT
   <213> 3.1.1: Human
<400> 4
<210> 5
   <211> 126
   <212> PRT
   <213> 21.4.1: Human
<400> 5
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asp Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Asn Arg Leu Arg Ser Asp Asp Thr Ala Val Tyr Tyr Cys
   85 90 95
   Ala Arg Asp Gln Pro Leu Gly Tyr Cys Thr Asn Gly Val Cys Ser Tyr
   100 105 110
   Phe Asp Tyr Trp Gly Gln Gly Thr Leu Val Thr Val Ser Ser
   115 120 125
<210> 6
   <211> 326
   <212> PRT
   <213> 21.4.1: Human
<400> 6
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Asn Phe Gly Thr Gln Thr
   65 70 75 80
   Tyr Thr Cys Asn Val Asp His Lys Pro Ser Asn Thr Lys Val Asp Lys
   85 90 95
   Thr Val Glu Arg Lys Cys Cys Val Glu Cys Pro Pro Cys Pro Ala Pro
   100 105 110
   Pro Val Ala Gly Pro Ser Val Phe Leu Phe Pro Pro Lys Pro Lys Asp
   115 120 125
   Thr Leu Met Ile Ser Arg Thr Pro Glu Val Thr Cys Val Val Val Asp
   130 135 140
   Val Ser His Glu Asp Pro Glu Val Gln Phe Asn Trp Tyr Val Asp Gly
   145 150 155 160
   Val Glu Val His Asn Ala Lys Thr Lys Pro Arg Glu Glu Gln Phe Asn
   165 170 175
   Ser Thr Phe Arg Val Val Ser Val Leu Thr Val Val His Gln Asp Trp
   180 185 190
   Leu Asn Gly Lys Glu Tyr Lys Cys Lys Val Ser Asn Lys Gly Leu Pro
   195 200 205
   Ala Pro Ile Glu Lys Thr Ile Ser Lys Thr Lys Gly Gln Pro Arg Glu
   210 215 220
   Pro Gln Val Tyr Thr Leu Pro Pro Ser Arg Glu Glu Met Thr Lys Asn
   225 230 235 240
   Gln Val Ser Leu Thr Cys Leu Val Lys Gly Phe Tyr Pro Ser Asp Ile
   245 250 255
   Ala Val Glu Trp Glu Ser Asn Gly Gln Pro Glu Asn Asn Tyr Lys Thr
   260 265 270
   Thr Pro Pro Met Leu Asp Ser Asp Gly Ser Phe Phe Leu Tyr Ser Lys
   275 280 285
   Leu Thr Val Asp Lys Ser Arg Trp Gln Gln Gly Asn Val Phe Ser Cys
   290 295 300
   Ser Val Met His Glu Ala Leu His Asn His Tyr Thr Gln Lys Ser Leu
   305 310 315 320
   Ser Leu Ser Pro Gly Lys
   325
<210> 7
   <211> 107
   <212> PRT
   <213> 21.4.1: Human
<400> 7
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Ile Tyr Ser Trp
               20                  25                  30
   Leu Ala Trp Tyr Gln Gln Lys Pro Gly Lys Ala Pro Asn Leu Leu Ile
           35                  40                  45
   Tyr Thr Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ile Phe Pro Leu
                   85                  90                  95
   Thr Phe Gly Gly Gly Thr Lys Val Glu Ile Lys
               100                 105
<210> 8
   <211> 107
   <212> PRT
   <213> 21.4.1: Human
<400> 8
<210> 9
   <211> 126
   <212> PRT
   <213> Human: 3.1.1H-A78T-V88A-V97A
<400> 9
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Lys Asp Gly Gly Asn Lys Tyr His Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Gly His Gln Leu Val Leu Gly Tyr Tyr Tyr Tyr Asn Gly
   100 105 110
   Leu Asp Val Trp Gly Gln Gly Thr Thr Val Thr Val Ser Ser
   115 120 125
<210> 10
   <211> 112
   <212> PRT
   <213> Human: 3.1.1L-L4M-L.83V
<400> 10
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Tyr Ser
               20                  25                  30
   Asn Gly Tyr Asn Phe Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Arg Thr Phe Gly Gln Gly Thr Lys Val Glu Ile Lys
               100                 105                 110

## Description

### BACKGROUND OF THE INVENTION

CD40 is a member of the tumor necrosis factor receptor (TNFR) superfamily. It is expressed on antigen presenting cells (B cells, dendritic cells, monocytes), hematopoietic precursors, endothelial cells, smooth muscle cells, epithelial cells, as well as the majority of human tumors. (Grewal & Flavell, Ann. Rev. Immunol., 1996, 16: 111-35; Toes & Schoenberger, Seminars in Immunology, 1998, 10(6) : 443-8). Studies using CD40 agonist agents have reported that stimulation of the CD40 receptor elicits a cascade of effects associated with anti-tumor activity. For example, stimulation of the CD40 receptor on antigen presenting cells has been shown to enhance their maturation, antigen-presenting function, costimulatory potential and their release of immunoregulatory cytokines (Lee et al., PNAS USA, 1999, 96(4) : 1421-6 ; Cella et al., J. Exp. Med., 1996, 184(2): 747-52). CD40 agonists have also been reported to promote the apoptosis of CD40+ tumors and enhance their ability to be processed by dendritic cells (von Leoprechting et al., Cancer Res., 1999, 59 :1287-94; Sotomayo et al., Nature Medicine,1999, 5(7): 780-87 ; Eliopoulos et al., Mol. Cell Biol., 2000, 29(15): 5503-15 ; Ziebold et al., Arch. Immunol. Therapiae Experimentalis, 2000, 48(4) :225-33 ; Hoffmann et al., J. Immunol., 2001, 24(2) : 162-71). The significance of these immune stimulatory and direct anti-tumor effects has been illustrated in animal models in which CD40 agonist antibodies have been shown to prevent tumor growth and reverse tumor tolerance (Diehl et al., Nature Med.,1999, 5(7) : 774-9; Francisco et al., Cancer Res., 2000, 60(12) : 32225-31). CD40 antibodies are referred to in the following patent publications: U.S. 5,786,456; U.S. 5,674,492; WO 02/088186; US 2003059427; US 20020142358; WO 01/56603; U.S. 5,801,227; EP 806963; WO 88/06891; and WO 94/04570. Formulations of proteins are described in WO02/30463. However, highly effective methods of administration and formulations for CD40 antibodies have not been described. Also useful would be a stable formulation suitable for use in such treatment.

### SUMMARY OF THE INVENTION

According to the present disclosure there is described a method of treating cancer in a patient in need of such treatment comprising administering to said patient a CD40 agonist antibody or a fragment thereof, wherein said antibody is administered according to an intermittent dosing regimen of at least two cycles, each cycle comprising (a) a dosing period during which a therapeutically effective amount of said CD40 agonist antibody is administered to said patient and thereafter (b) a resting period. In one embodiment of the disclosure, the administration produces a plasma concentration of the antibody of 0.01 µg/ml to 10 µg/ml for at least three hours and the resting period is for at least 1 week. According to the present disclosure there is described, the dosing period is for at least one day, 1-5 days, or 1-3 days. In other embodiments, the resting period is from 1-8, 1-6 weeks, 2-5 weeks, or 3-4 weeks.

According to the present disclosure there is described, the therapeutically effective amount of the CD40 agonist antibody produces the plasma concentration of said antibody of about 0.03 µg/ml to 10 µg/ml, about 0.03 µg/ml to 1 µg/ml, about 0.03 µg/ml to 0.3 µg/ml, or about 0.1 µg/ml to 0.3 µg/ml for 3 to 120 hours. According to the present disclosure there is described, the specified plasma concentration is maintained for at least one day, 24 to 30 hours, 24 to 36 hours, 24 to 48 hours, 24 to 72 hours, 24 to 96 hours, or 24 to 120 hours. In some embodiments, the plasma concentration is maintained for 3 to 96 or 12 to 72 hours.

According to the present disclosure there is described, the therapeutically effective amount of the CD40 agonist antibody administered during the dosing period is about 0.03 to 3.0 mg/kg/day, 0.1 to 3.0 mg/kg/day, 0.1 to 1.0 mg/kg/day, or about 0.1 to 0.3 mg/kg/day. According to the present disclosure there is described the dosage is administered for 1-5 days or 1-3 days, either consecutively or on alternate days.

The intermittent dosing regimen of CD40 agonist antibodies, as described above in connection with tumor treatment, is also useful in enhancing immune responses in patients and such use, therefore, is also disclosed. According to the present disclosure there is described, the enhancement of a patient's immune response results in increased CD23 or MHC-II expression on B-cells in patient's whole blood, which, for example, may be measured at the end of a dosing period.

According to the present disclosure there is described, the anti-CD40 antibody is administered to a patient who suffers from primary and/or combined immunodeficiencies, including CD40- dependent immunodeficiency with Hyper-IgM syndrome, Common Variable Immunodeficiency, Bruton's Agammaglobulinemia, IgG subclass deficiencies, and X-linked SCID (common gamma chain mutations). According to the present disclosure there is described, the anti-CD40 antibody is administered to treat a patient who is immunosuppressed, for example due to chemotherapy, or has an immune-debilitating disease, including any acquired immune deficiency disease, such as HIV. According to the present disclosure there is described, the anti-CD40 antibody is administered to enhance the immunity of an elderly patient. According to the present disclosure there is described, the anti-CD40 antibody is administered to treat a patient who has a bacterial, viral, fungal or parasitic infection According to the present disclosure there is described, a human agonist anti-CD40 antibody may be administered prophylactically to a patient who, because of age, illness or general poor health is susceptible to infection to prevent or to reduce the number or severity of infections.

The present disclosure also describes a method of treating a tumor in a patient comprising administering a CD40 agonist antibody and a DNA replication inhibitor, preferably a platin-derivative, especially cisplatin. According to the present disclosure there is described, cisplatin is administered intravenously. According to the present disclosure there is described, cisplatin is administered in an amount of from about 25 to 300 mg per m², about 50 to 150 mg per m², or about 75 to 100 mg per m² of the patient's body surface area. According to the present disclosure there is described, the cisplatin is administered in one dose (e.g., a single intravenous infusion). In another embodiment, it is administered over 2-5 days. In certain embodiments, the amount of the CD40 antibody being administered in combination with cisplatin is administered in a dosage of about 0.1 to 3.0 mg/kg, or about 0.1 to 1.0 mg/kg, or about 0.1 to 0.3 mg/kg.

According to the present disclosure there is described, administration of cisplatin is combined with the intermittent dosing regimen of the CD40 antibody, with cisplatin being administered during one or more of the dosing periods or rest periods.

According to the present disclosure there is described, the invention relates to a method of treating a tumor in a patient in need of such treatment by administering to the patient a CD40 agonist antibody or a fragment thereof in a dosage of less than 1 mg/kg/day, wherein the Cₘₐₓ serum concentration in the patient resulting from administration of the antibody is less than 50 µg/ml. In one embodiment, the dosage is between 0.1 to 0.3 mg/kg and the Cₘₐₓ serum concentration of the antibody in the patient is between 0.5 and 10 µg/ml.

The present invention relates to a stable liquid pharmaceutical formulation suitable for parenteral administration comprising an anti-CD40 antibody at a pH of from 5.0-6.0 and a pharmaceutically acceptable carrier, the formulation being stable for a period of at least three months. The formulation preferably has a concentration of CD40 antibody of at least about 5 mg/ml. In one embodiment, the formulation comprises an anti-CD40 antibody, sodium acetate, sodium chloride, and polysorbate 80. Preferably, it comprises 20 mM sodium acetate, 140 mM sodium chloride, and 0.2 mg/mL polysorbate 80. The anti-CD40 antibody preferably has the amino acid sequence of an antibody selected from the group consisting of antibody 21.4.1 or according to the disclosure 3.1.1.

### BRIEF DESCRIPTION OF THE DRAWINGS

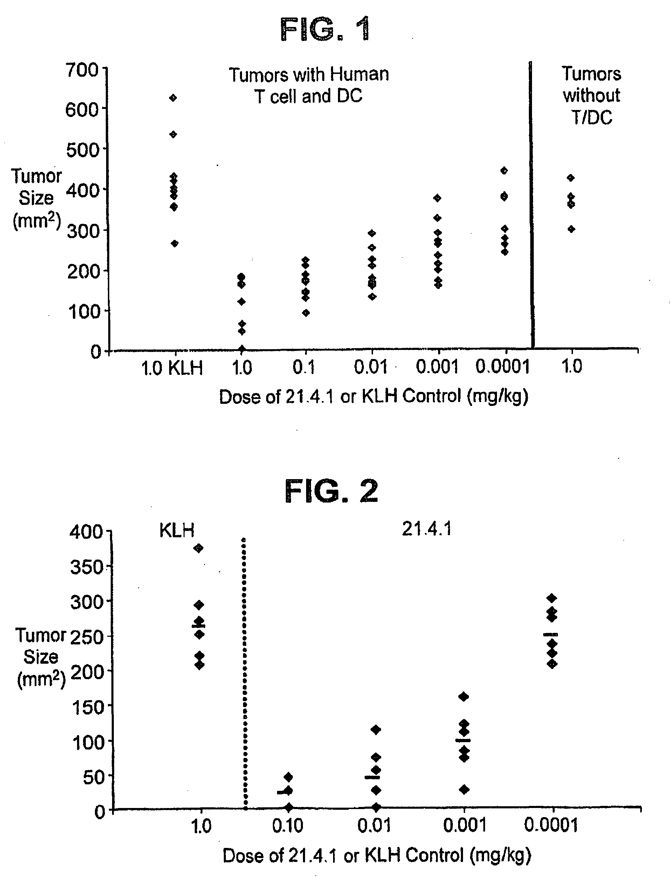

Figure 1 shows inhibition of growth of the CD40(-) Tumor K562 by a CD40 agonist antibody in the presence of immune cells. Animals received a single injection (IP) of 21.4.1 or KLH at the time of tumor challenge. Tumor size is reported for each individual animal on Day 21 in mm² (10 animals per group). The study is representative of at least 5 separate studies.
Figure 2 shows inhibition of growth of the human breast tumor cell line BT 474 by a CD40 agonist antibody. The values represent individual tumor measurements taken on Day 53 after injection using 6 animals per group. The study is representative of two separate experiments. The mean for each treatment group is indicated by the horizontal line.

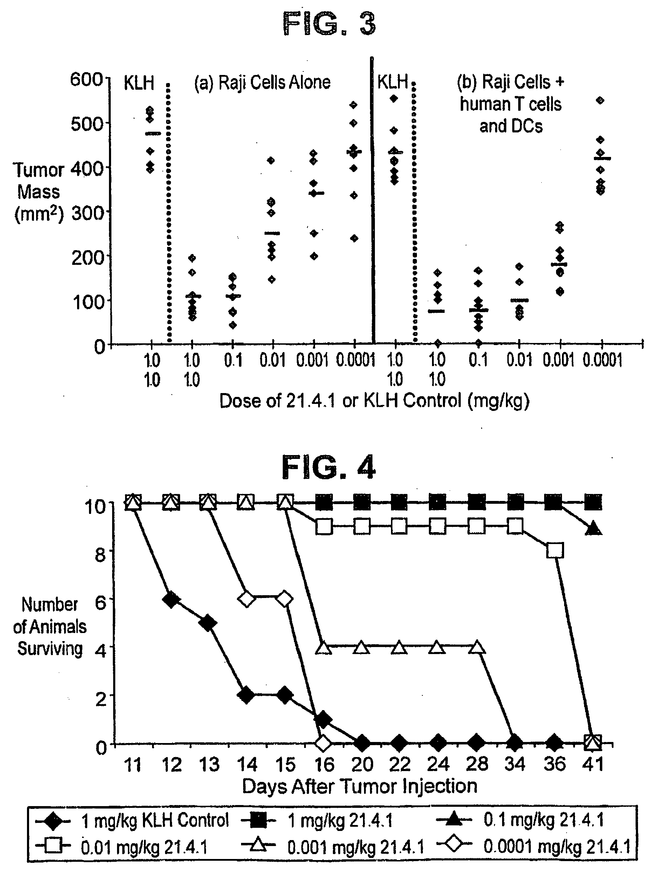

Figure 3 shows inhibition of CD40(+) tumor growth by a CD40 agonist antibody, alone or in the presence of immune cells. Animals received a single injection of 21.4.1 at the time of tumor challenge. (a) Tumors were injected alone or (b) together with human peripheral blood T cells and DC. The data points represent the tumor size (mm²) for each individual animal. The mean for each treatment group (N=10) is indicated by the horizontal line. The study is representative of at least 3 separate experiments.
Figure 4 shows effects of a CD40 agonist antibody in delaying mortality induced by a B cell Lymphoma (Daudi). The data points refer to the mean number of surviving animals, N=10 per group.

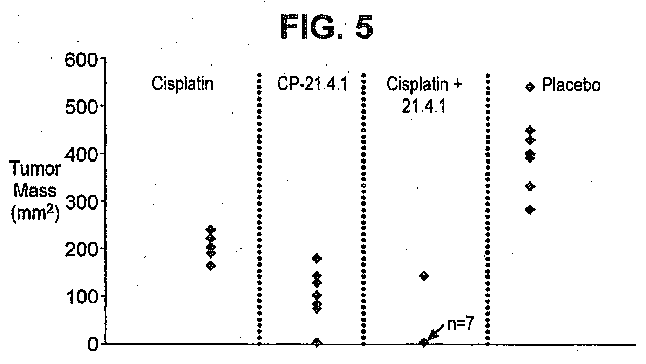

Figure 5 shows tumor regression caused by a combination therapy with a CD40 agonist antibody and cisplatin.

### DETAILED DESCRIPTION OF THE PREFERRED EMBODIMENTS

As used herein, the term "agonist CD40 antibody" or "agonist anti-CD40 antibody" means an antibody that specifically binds to human CD40 molecule and increases one or more CD40 activities by at least about 20% when added to a cell, tissue or organism expressing CD40. In some embodiments, the antibody activates CD40 activity by at least 40%, 50%, 60%, 70%, 80%, or 85%. In some embodiments, the activation occurs in the presence of CD40L. In some embodiments, the activity of the activating antibody is measured using a whole blood surface molecule upregulation assay. In another embodiment, the activity of the activating antibody is measured using a dendritic cell assay to measure IL-12 release. In another embodiment the activity of the activating antibody is measured using an *in vivo* tumor model.

The term "antibody" as used herein refers to an intact antibody, or a binding fragment thereof that competes with the intact antibody for specific binding. Binding fragments are produced by recombinant DNA techniques, or by enzymatic or chemical cleavage of intact antibodies. Binding fragments include Fab, Fab', F(ab')₂, Fv, and single-chain antibodies. It is understood that reference to an intact (*e.g*., whole, full-length, etc.) antibody herein includes an antibody having a terminal lysine deletion in the heavy chain, which commonly occurs during recombinant expression.

Preferably, the agonist CD40 antibody is a human antibody. As used herein, the term "human antibody" means an antibody in which the variable and constant domain sequences are derived from human sequences. Human CD40 antibodies are described in detail in U.S. provisional application no. 60/348,980, filed November 9, 2001, and PCT International Application No. PCT/US02/36107 (now published as WO 03/040170) filed November 8, 2002,. Human antibodies provide a substantial advantage in the treatment methods of the present disclosure, as they are expected to minimize the immunogenic and allergic responses that are associated with use of non-human antibodies in human patients.

Exemplary human anti-CD40 antibodies useful for the present invention include antibodies having the amino acid sequences of antibodies designated 21.4.1; antibodies designated 3.1.1, 3.1.1H-A78T-V88A-V97A/3.1.1L-L4M-L83V, 3.1.1 H-A78T, 3.1.1 HA78T-V88A-V97A, 7.1.2, 10.8.3, 15.1.1, 21.2.1, 22.1.1, 22.1.1H-C109A, 23.5.1, 23.25.1, 23.28.1, 23.28.1 H-D16E, 23.29.1, 24.2.1, and 23.28.1 L-C92A are also disclosed herein.

Antibodies that recognize the same or similar epitopes, or a portion thereof, as any of the exemplary antibodies are hereby disclosed. That is, as would be understood by one skilled in the art based upon the disclosure provided herein, an antibody that competes with an antibody of the invention and disclosure *(e.g.,* 3.1.1, 3.1.1H-A78T, 3.1.1H-A78T-V88A-V97A, 7.1.2, 10.8.3, 15.1.1, 21.4.1, 21.2.1, 22.1.1, 22.1.1H-C109A, 23.5.1, 23.25.1, 23.28.1, 23.28.1 H-D16E, 23.29.1, 24.2.1, 3.1.1H-A78T-V88A-V97A/3.1.1L-L4M-L83V and 23.28.1L-C92A, and the like) can be useful as disclosed elsewhere herein. An antibody of interest that competes with an antibody exemplified herein can be readily identified using methods well known in the art for the characterization of antibodies. More specifically, assays for assessing the binding characteristics of an antibody, as well as for comparing those binding characteristics to those of another antibody, are well known in the art. Such methods include, but are not limited to, ELISA-based assays, use of BIAcore binding studies, as well as those detailed in US Patent Application Publication No. 2003/0157730A1 to Walker et al.

By the term "compete", as used herein with regard to an antibody, is meant that a first antibody competes for binding with a second antibody where binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope or ligand, whether to the same, greater, or lesser extent, the antibodies are said to "cross-compete" with each other for binding of their respective epitope(s). For instance, cross-competing antibodies can bind to the epitope, or portion of the epitope, to which the antibodies of the invention and disclosure (*e.g*., 3.1.1, 3.1.1H-A78T, 3.1.1H-A78T-V88A-V97A, 7.1.2, 10.8.3, 15.1.1, 21.4.1, 21.2.1, 22.1.1, 22.1.1H-C109A, 23.5.1, 23.25.1, 23.28.1, 23.28.1H-D16E, 23.29.1, 24.2.1, 3.1.1H-A78T-V88A-V97A/3.1.1L-L4M-L83V and 23.28.1 L-C92A) bind. Both competing and cross-competing antibodies are hereby disclosed.

In addition the exemplary antibodies may be further modified by substitution, addition or deletion of one or more amino acid residues without eliminating the antibody's ability to bind the antigen and expert its agonistic function. Indeed, an antibody designated "3.1.1H-A78T-V88A-V97A/3.1.1L-L4M-L83V", comprises three amino acid substitutions in the heavy chain variable region, *i.e.,* a substitution from alanine to threonine at amino acid residue number 78, a substitution from valine to alanine at amino acid residue number 88, and a substitution from valine to alanine at amino acid residue number 97 (SEQ ID NO:9), all with respect to the amino acid sequence of the heavy chain variable region of antibody 3.1.1 (SEQ ID NO:1). In addition, the 3.1.1H-A78T-V88A-V97A/3.1.1L-L4M-L83V antibody further comprises an amino acid substitution from leucine to methionine at amino acid residue number 4 and a substitution from leucine to valine at amino acid residue number 83 in the light chain variable region (SEQ ID NO:10) compared with the amino acid sequence of the variable region of the light chain of antibody 3.1.1 (SEQ ID NO:3). The amino acid sequences of the constant regions of the heavy chains (SEQ ID NO:2) and light chains (SEQ ID NO:4) of 3.1.1 and 3.1.1H-A78T-V88A-V97A/3.1.1L-L4M-L83V antibodies are the same. Antibody 3.1.1H-A78T-V88A-V97A/3.1.1L-L4M-L83V is also referred to as "3.1.1H3L2" to reflect that the antibody comprises three amino acid substitutions in the heavy chain and two amino acid substitutions in the light chain relative to antibody 3.1.1.

Thus, the disclosed antibodies may be modified by substitution, addition, or deletion of one to ten, one to five, or one to three amino acid residues, e.g., in a CDR or framework region. The exemplary antibodies and methods of producing them are described in detail in U.S. provisional application no. 60/348,980, filed November 9, 2001, and PCT International Application No. PCT/US02/36107 (WO 03/040170), filed November 8, 2002.

Hybridomas 3.1.1, 7.1.2, 10.8.3, 15.1.1 and 21.4.1 were deposited in accordance with the Budapest Treaty, in the American Type Culture Collection (ATCC), 10801 University Boulevard, Manassas, VA 20110-2209, on August 6, 2001. Hybridomas 21.2.1, 22.1.1, 23.5.1, 23.25.1, 23.28.1, 23.29.1 and 24.2.1 were deposited in the ATCC on July 16, 2002. The hybridomas have been assigned the following deposit numbers:

| Hybridoma | Deposit No. |
|---|---|
| 3.1.1 (LN 15848) | PTA-3600 |
| 7.1.2 (LN 15849) | PTA-3601 |
| 10.8.3 (LN 15850) | PTA-3602 |
| 15.1.1 (LN 15851) | PTA-3603 |
| 21.4.1 (LN 15853) | PTA-3605 |
| 21.2.1 (LN 15874) | PTA-4549 |
| 22.1.1 (LN 15875) | PTA-4550 |
| 23.5.1 (LN 15855) | PTA-4548 |
| 23.25.1 (LN 15876) | PTA-4551 |
| 23.28.1 (LN 15877) | PTA-4552 |
| 23.29.1 (LN 15878) | PTA-4553 |
| 24.2.1 (LN 15879) | PTA-4554 |

The sequences of these antibodies are known, and described in WO 03/040170. For convenience, the amino acid sequences of heavy and light chains of two of these antibodies are shown below:
Antibody 3.1.1:

| | |
|---|---|
| 3.1.1: Heavy Chain Protein Sequence | Variable (SEQ ID NO:1): |
| | |
| | Constant (SEQ ID NO:2): |
| | |
| 3.1.1: Light Chain Protein Sequence | Variable (SEQ ID NO:3): |
| | |
| | Constant (SEQ ID NO:4): |
| | |

| | |
|---|---|
| 3.1.1H-A78T-V88A-V97A: Heavy Chain Protein Sequence | Variable (SEQ ID NO:9): |
| | |
| | Constant (SEQ ID NO:2): |
| | |
| 3.1.1 L-L4M-L83V: Light Chain Protein Sequence | Variable (SEQ ID NO:10): |
| | |
| | Constant (SEQ ID NO:4): |
| | |

Antibody 21.4.1:

| | |
|---|---|
| 21.4.1: | Variable (SEQ ID NO:5): |
| Heavy Chain Protein Sequence | |
| | Constant (SEQ ID NO:6): |
| | |
| 21.4.1: | Variable (SEQ ID NO:7): |
| Light Chain Protein Sequence | |
| | Constant (SEQ ID NO:8): |
| | |

Thus, the amino acid sequence of 21.4.1 antibody comprises the amino acid sequences set forth in SEQ ID NOs:5-8, the amino acid sequence of 3.1.1. antibody comprises the amino acid sequences set forth in SEQ ID NOs:1-4, and the amino acid sequence of 3.1.1H-A78T-V88A-V97A/3.1.1L-L4M-L83V antibody comprises the sequences set forth in SEQ ID NO:9, SEQ ID NO:2, SEQ ID N010 and SEQ ID N04. The amino acids which differ between 3.1.1 and 3.1.1H-A78T-V88A-V97A/3.1.1L-L4M-L83V are underlined.

It would be understood, based upon the disclosure provided herein, that a 3.1.1 antibody of the invention encompasses any combination of the heavy and/or light variable regions set forth herein. That is, an antibody can comprise any combination of variable regions, including, but not limited to, 3.1.1 H (SEQ ID NO:1)/3.1.1L (SEQ ID NO:3), 3.1.1 H (SEQ ID NO:1)/3.1.1L-L4M-L83V (SEQ ID NO:10), 3.1.1 H-A78T-V88A-V97A (SEQ ID NO:9)/3.1.1L (SEQ ID NO3), and, more preferably, 3.1.1H-A78T-V88A-V97A (SEQ ID NO:9)/3.1.1L-L4M-L83V (SEQ ID NO:10).

According to the present disclosure tumor treatment inhibits cancer cell proliferation, inhibits or prevents an increase in tumor weight or volume, and/or causes a decrease in tumor weight or volume. In some embodiments, the tumor treatment prolongs patient survival. According to the present disclosure, tumor growth is inhibited at least 50%, 55%, 60%, 65%, 70% or 75%, compared to those not treated. In some embodiments, the tumor is CD40 positive. According to the present disclosure, the tumor is CD40 negative. The tumor can be a solid tumor or a non-solid tumor such as lymphoma. According to the present disclosure, an anti-CD40 antibody is administered to a patient who has a tumor that is cancerous

Patients that can be treated with anti-CD40 antibodies or antibody portions include, but are not limited to, patients that have been diagnosed as having brain cancer, lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, gastric cancer, colorectal cancer, colon cancer, gynecologic tumors (e.g., uterine sarcomas, carcinoma of the fallopian tubes, carcinoma of the endometrium, carcinoma of the cervix, carcinoma of the vagina or carcinoma of the vulva), cancer of the esophagus, cancer of the small intestine, cancer of the endocrine system (e.g., cancer of the thyroid, parathyroid or adrenal glands), sarcomas of soft tissues, leukemia, myeloma, multiple myeloma, cancer of the urethra, cancer of the penis, prostate cancer, chronic or acute leukemia, solid tumors of childhood, Hodgkin's disease, lymphocytic lymphomas, non-Hodgkin lymphoma, cancer of the bladder, liver cancer, renal cancer, cancer of the kidney or ureter (e.g., renal cell carcinoma, carcinoma of the renal pelvis), or neoplasms of the central nervous system (e.g., primary CNS lymphoma, spinal axis tumors, brain stem gliomas or pituitary adenomas), glioma or fibrosarcoma.

As used herein, the term "patient" refers to a human or a non-human mammal that expresses a cross-reacting CD40 (e.g., a primate, cynomolgus or rhesus monkey). Preferably a patient being treated is human.

As used herein, the term "intermittent dosing regimen" means a dosing regimen that comprises administering a CD40 agonist antibody, followed by a rest period.

As used herein, the term "resting period" means a period of time during which the patient is not given a CD40 agonist antibody. For example, if the antibody has been given on a daily basis, there would be rest period if the daily administration is discontinued, e.g., for some number of days or weeks. If a dose is administered on a different schedule a rest period would occur where that dosing is discontinued for some time. Alternately, a rest period may occur where the concentration of the antibody is maintained at a sub-therapeutic level.

According to the present disclosure, the antibody is not given after the second rest period, i.e., when the method of the disclosure involves two cycles, the drug need not be administered following the second rest cycle.

Preferably, during the rest period, the plasma concentration of the antibody is maintained at sub-therapeutic level.

The dosing period and/or the dose of the antibody can be the same or different between cycles.

The total treatment time (i.e., the number of cycles for treatment) will vary from patient to patient based on sound medical judgment and factors particular to the patient being treated. In general, the treatment is administered until a satisfactory response is obtained. In certain embodiments of the disclosure, the treatment period comprises 2-20, 2-15, 2-10, 2-7, 2-5 cycles or 2-3 cycles.

The antibody may be administered by any means desired, including, e.g., intravenous, subcutaneous, intramuscular, parenteral, intratumor, and transdermal administration. In one embodiment the CD40 antibody is administered intravenously. In another, it is administered using a microneedle device; such devices are well known and include, e.g., the device described in WO 03/084598.

When administered in combination with a DNA replication inhibitor, e.g., cisplatin, the antibody may be administered before, during, or after administration of the inhibitor.

In one aspect, the invention relates to an aqueous solution for intravenous injection, with the pH of about 5.0 to 6.0, preferably pH of about 5.5. Such a solution may be formulated with sodium acetate (trihydrate), acetic acid (glacial), Polysorbate 80, sodium chloride and water. It is preferred that the antibody solution be stored at refrigerated temperatures between 2° C and 8° C, and not be frozen.

Also disclosed are methods of treating a tumor in a patient in need of such treatment comprising administering to said patient a combination of a therapeutically effective amount of a CD40 agonist antibody and a therapeutically effective amount of a DNA replication inhibitor, e.g., a platin-derivative. According to the present disclosure, a CD40 agonist antibody works in synergistic combination with the platin-derivative compound, especially cisplatin, such that anti-tumor effect of the combination is greater than what would be predicted from administration of each compound alone.

Platin-derivatives are well-known group of compounds that exhibit their anti-tumor activity by interfering with DNA replication. According to the present disclosure, platin derivatives are selected from the group consisting of cisplatin (cis-diaminedichloroplatinum, See Merck Index), carboplatin and oxaliplatin.

The invention will be described by reference to the following examples.

### EXAMPLES

### Example 1: Effects Of Antibody On Lymph Node Cells From Cancer Patients

Effects of a human anti-CD40 antibody (21.4.1) on lymph node cells obtained from cancer patients stimulated with autologous tumor cells was examined.

Lymph node cells and tumors were collected from patients with renal cell carcinoma, non-small cell lung cancer, transitional cell carcinoma of the bladder, colon cancer, prostate cancer, and head and neck cancer. The lymph node cells were placed into culture together with irradiated collagenase treated tumors (recovered from the same patient) in the presence or absence of 21.4.1 (1 µg/mL; 6.7 nM). Proliferation was assessed using ³H-thymidine 96 hours later. The number of INFγ producing cells was assessed by ELISPOT, following restimulation.

The antibody enhanced the number of IFNγ+ positive T cells in cultures of lymph node cells stimulated with tumor antigen. Further, the proliferation of these lymph node cells in response to tumor antigen was enhanced 3-4 fold.

The antibody enhanced the proliferation and cytokine producing capacity of lymph node cells obtained from cancer patients when stimulated with tumor antigen.

### Example 2 : Binding of Antibody to Fc Receptor

The binding of an anti-CD40 antibody (21.4.1) to Fc receptors on human and cynomolgus leukocytes was examined.

Flow cytometric studies indicated that FcR types FcγRII (CD32) and FcγRIII (CD16). as well as very low levels of FcγRI (CD64), were expressed on human leukocytes. The binding of 21.4.1 to Fc receptors (FcR) on human or cynomolgus peripheral blood leukocytes was determined by using ¹²⁵I-21.4.1 and a human IgG1 control mAb. Human leukocytes from normal donors or cynomolgus leukocytes were Isolated from whole blood using plasma gel and washed thoroughly to allow dissociation of receptor-bound serum immunoglobulins. Centrifugation through a sucrose cushion was used to separate cell-bound and free antibodies. Studies were performed at 4°C in the presence of sodium azide to prevent receptor internalization.

21.4.1 was tested for specific binding to FcR by using excess unlabeled human IgG2 Isotype matched antibody as a competitor. ¹²⁵I-21.4.1 specific binding to FcR on human leukocytes (n=5 donors) was -1.0±8.5%, and specific binding to FcR on cynomolgus leukocytes (n=4) was 15±13%. Addition of 500-fold excess unlabeled 21.4.1, which would block any specific binding of ¹²⁵I-21.4.1 to leukocyte CD40 receptors as well as FcR, demonstrated 49% and 67% specific binding of ¹²⁵I-21.4.1 to CD40 receptors on human and cynomolgus leukocytes, respectively (% specific binding to CD40 was calculated by subtracting % binding to FcR from total % specific binding). As a control, ¹²⁵I-IgG1 consistently demonstrated specific binding to human and cynomolgus leukocytes. The specific binding of the IgG1 control antibody to FcR on human and cynomolgus leukocytes accounted for 56% and 51% of the total bound radioactivity, respectively.

These studies indicate that the antibody shows minimal specific binding to Fc receptors on human and cynomolgus leukocytes.

### Example 3 : Whole-Blood Cytokine Release Assay

An anti-CD40 antibody (21.4.1) was tested for its ability to induce the release of cytokines from unstimulated human whole blood using an in vitro whole blood assay which correlates with induction of antibody-mediated cytokine release in humans. 21.4.1 was tested at 1, 10 and 100 µg/mL, along with a murine anti-human CD3 IgG1 as a positive control that induces cytokine release through an Fc mediated pathway, and LPS as a second positive control that induces cytokines by stimulating macrophages. The donors used included individuals that responded to both the murine antibody and LPS (4 donors), as well as individuals who only responded to the LPS (3 donors). Heparinized whole blood was cultured with 21.4.1 for 5 hours and plasma was collected and analyzed for tumor necrosis factor alpha (TNF-α), interferon gamma (INF-γ) and interleukin-6 (IL-6) by ELISA (using commercially available kits). Cultures were also incubated for 48 hours and analyzed for interleukin-1 beta (IL-1β).

Cytokines were not detected in the plasma of human blood cultured with 1 or 10 µg/mL 21.4.1. Only one donor treated with 100 µg/mL of the antibody showed low but measurable levels of two cytokines (34 pg/mL of TNF-q in and 90 pg/mL IL-6). This donor was re-tested subsequently and showed no detectable induction of TNF-α or IL-6. There was no elevation of INFγ or IL-1β in any of the samples.

These studies indicate that 21.4.1 does not induce inflammatory cytokines in human whole blood.

### Example 4 : Pharmacodynamics and Pharmacokinetics of Antibody

A CD40 antibody (21.4.1) was administered intravenously at various doses (1 mg/kg n = 4, 3 mg/kg n = 4, 5 mg/kg n = 2 and 10 mg/kg n = 2) to cynomolgus monkeys. Heparinized blood was drawn from the monkeys at various time points pre- and postdose. The blood was aliquoted and stained. Data were acquired using a Becton Dickinson FACSCalibur and analyzed with CellQuest software. Results were calculated as fold increases in median fluorescence intensity as compared to pre-dose values.

MHC Class II expression, reflecting activation state and antigen presenting capacity of B-cells, increased by 2.5 to 3 fold by 24 hours after dosing for all doses tested, with no clear dose-response relationship observed. CD23 expression, another marker of B-cell activation, was evaluated in 2 animals at 3 mg/kg, and one animal at 10 mglkg. CD23 expression increased ≥20-fold at 24 hours after dosing with no dose effect observed. Upregulation of both surface markers persisted (≥2-fold increase) while 21.4.1 levels remained above 1 µg/ml CD71 (transferrin receptor) and CD86 costimulatory molecule levels alsSo showed moderate upregulation, while CD80 expression did not change significantly.

21.4.1 upregulates surface markers in cynomolgus B-cells in vivo. MHC Class II and CD23 expression on CD20+ cells increase with treatment, and 1 mg/kg (corresponding to a Cₘₐₓ of ~ 20 µg/mL and an exposure of ≥0.1 µg/mL for 4 days) appears to produce a saturating pharmacodynamic response in cynomolgus B-cells. The duration of this response was longer at higher doses.

The pharmacokinetic properties of an antl-CD40 antibody (21.4.1) were examined in cynomolgus monkeys following intravenous (IV) administration of a single dose of 1, 3, 5 or 10 mg/kg. 21.4.1 was characterized by low systemic clearance (0.0133 to 0.0635 mL/min/kg) and small volume of distribution at steady state (0.0459 to 0.0757 L/kg), resulting in an apparent mean elimination half-life of 0.75 to 2.0 days (Table 1). The pharmacokinetics of 21.4.1 appeared to be dose-dependent over the dose range examined. Clearance values generally decreased with increasing dose from 1 to 10 mg/kg. and the apparent mean elimination half-life increased from 0.75 day at 1 mg/kg to 2.0 days at 10 mg/kg. The volume of distribution at steady state was similar at different doses (mean of 0.0575 L/kg).

The observed dose-dependent clearance may be in part due to the binding of 21.4.1 to CD40 receptors that are widely expressed in normal tissues and the subsequent internalization and elimination of the antibody-receptor complex. Development of primate anti-human antibody (PAHA) response may also contribute to the accelerated clearance in some monkeys. PAHA was evaluated only after individual serum concentrations of 21.4.1 reached the lower limit of quantitation (LLOQ, 0.03 µg/mL) since the presence of 21.4.1 in test serum interferes with the assay for PAHA. Anti-21.4.1 antibodies were detected in all monkeys in the 3, 5, and 10 mg/kg dose groups at 14 to 28 days following administration of the antibody.

**Table 1**

| Mean (± SD) Pharmacokinetic Parameters of 21.4.1 in Cynomolgus Monkeys Following a Single IV Administration at 1, 3, 5, and 10 mg/kg | | | | | |
|---|---|---|---|---|---|
| Dose | N/ | CL | Vdss | t_{1/2} | AUC_{(0-∞)} |
| (mg/kg) | Gender | (mL/min/kg) | (L/kg) | (day) | (µgh/mL) |
| 1 | 2/sex | 0.0635 ± 0.0245 | 0.0757 ± 0.0265 | 0.75 ± 0.21 | 298 ± 126 |
| 3 | 2/sex | 0,0213 ± 0.0055 | 0.0459 ± 0.0055 | 1.4 ± 0.3 | 2460 ± 600 |
| 5 | 2F | 0.0174 | 0.0488 | 1.4 | 4790 |
| 10 | 1/sex | 0.0133 | 0.0529 | 2.0 | 12500 |

### Example 5: Anti-Tumor Activity of Antibody

The tumor growth inhibitory activity of a CD40 antibody (21.4.1) was determined in SCID-beige mice injected SC with tumor cells alone (1 X 10⁷) or with human DC (1 X 10⁵) and T cells (5 X 10⁵) from the same donor. The ratio of tumor cells to DC and T cells was 100:1:5. Unless otherwise indicated, the results are presented in terms of the tumor size in mm² at one fixed time point pre-determined (from kinetic experiments) to be the time when tumor growth in control animals reached a size of 300-400 mm² and it was no longer humane to continue the experiment. In all cases, only one injection of 214.1 was administered which had a T_{1/2} of >30 days In SCID-beige mice.

### Example 5(a): Effects of Antibody on CD40(-) Human Tumors

The effects of a CD40 antibody (21.4.1) on the growth of CDR40(-) tumors (e.g., erythroleukemia and colon carcinoma) were examined. In particular, K562 tumors were chosen to assess the efficacy of 21.4.1 against a CD40(-) low immunogenic (class I and II negative) tumor

SCID-beige mice were injected SC with the CD40(-) erythroleukemic tumor, K562 (ATCC CCL-243) alone or in the presence of human peripheral blood T cells and DCs. Animals received a single IP injection of 21.4.1 either at the time of tumor injection or 5 days later using various dose levels.

A single IP injection of 21.4.1 resulted in the dose-dependent inhibition of K562 tumor growth when immune cells were present as illustrated on Day 21 after tumor challenge (Figure 1). The amount of 21.4.1 to cause a 50% inhibition of tumor growth was 0.005 mg/kg corresponding to a Cₘₐₓ serum concentration of 0.05 µg/mL. Similar results were observed with the CD40(-) colon carcinoma, Lovo (ATCC CCL-229). The results were identical when 21.4.1 was administered on Day 0 or Day +5 relative to tumor challenge. The growth of these CD40(-) tumors was not inhibited by 21.4.1 in the absence of immune cells.

21.4.1 prevents the growth of CD40(-) tumors when immune cells are present, suggesting enhancement of immune mediated anti-tumor activity. This was demonstrated against a colon carcinoma and an erythroleukemic tumor. This anti-tumor activity was also demonstrated using antibody 3.1.1 for the colon carcinoma and for 3.1.1H-A78T-V88A-V97A/3.1.1L-L4M-L83V antibody (IC50 < 0.01 mg/kg) in the erythroleukemic tumor. Thus, the data disclosed herein demonstrate that 3.1.1H.A78T-V88A-V97A/3.1.1L-L4M-L83V antibody has the *in vivo* activity of 3..1.1 antibody. These *in vivo* tumor results further support that given the similar *in vitro* data obtained where the two antibodies were compared, antibody 3.1.1 and antibody 3.1.1H-A78T-V88A-V97A/3.1.1L-L4M-L83V will perform in a similar manner *in vivo.* Thus, results obtained using 3.1.1 apply to 3.1.1H-A78T-V88A-V97A/3.1.1L-L4M-L83V in this and other assays.

### Example 5(b): Effects of Antibody on Human Breast and Prostate Tumor Growth

The effects of an anti-D40 antibody (21.4.1) on preventing the growth of breast and prostate tumors was examined.

SCID-beige mice were challenged with the human breast tumor, BT 474 (ATCC HTB-20), SC, together with human peripheral blood T cells and DC. Animals received a single dose of 21.4.1 (IP) at the time of tumor injection.

As shown in Figure 2, a single injection of 21.4.1 prevented the growth of BT 474 cells in the presence of immune cells. The amount of 21.4.1 necessary to cause a 50% reduction in tumor growth was 0.005 mg/kg corresponding to a Cₘₐₓ serum concentration of 0,05 µg/mL. Similar results were observed against the human prostate cancer cell line, PC-3 (ATCC CRL-1435). This was also demonstrated using antibody 3.1.1 and can be expected for 3.1.1 H-A78T-V88A-V97A/3 1.1L-L4M-L83V.

### 21.4.1 prevents the growth of human breast and prostate tumors.

### Example 5(c): Anti-Tumor Effects of Antibody on CD40(+) Tumors

The effects of an anti-CD40 antibody (21.4.1) on anti-tumor activity against CD40(+) tumors and changes in efficacy in the presence and absence of immune cells was studied.

SCID-beige mice were injected subcutaneously with the CD40(+) Raji B cell lymphoma (ATCC CCL-86) (SC) followed by a single dose of 21.4.1 (IP) at the time of tumor injection. Some animals were also injected with human T cells and DC. Tumor growth was assessed on Day 21.

As shown in Figure 3, the amount of 21.4.1 to cause a 50% inhibition of tumor growth in the absence of immune cells was 0.02 mg/kg, corresponding to a Cₘₐₓ serum concentration of 0.2 µg/mL. When tumor cells were co-injected with immune cells, the amount of 21.4.1 necessary to cause a 50% inhibition of tumor growth was decreased 20-fold to 0.001 mg/kg (Cₘₐₓ serum concentration = 0.01 µg/mL).

These results illustrate that 21.4.1 has direct anti-tumor activity against CD40(+) tumors. This observation was also made for 3.1.1H-A78T-V88A-V97A/3.1.1L-L4M-L83V (IC50 < 0.01 mg/kg), This anti-tumor activity for antibody 21.4.1 was enhanced when immune cells were present and this was also demonstrated with antibody 3.11 and is expected for antibody 3.1.1H-A78T-V88A-V97A/3.1.1L-L4M-L83V.

### Example 5(d): Anti-Tumor Effects of Antibody on B-Cell Lymphoma

The ability of an anti-CD40 antibody according to the invention (21.4.1) to delay mortality in a CD40(+) systemic tumor model using a B cell lymphoma was assessed.

SCID-beige mice were injected IV with the B cell lymphoma Daudi (ATCC CCL-213). 21.4.1 was administered as a single injection (IP) at the time of tumor injection Mortality was monitored for 58 days.

As shown in Figure 4, a single injection of 21.4.1 prevented mortality induced by a systemically administered tumor cell line.

21.4.1 delays mortality in a CD40(+) systemic tumor model using a B cell lymphoma. This was also demonstrated using 3.1.1 and similar results are expected for 3.1.1H-A78T-V88A-V97A/3.1.1 L-L4M-L83V.

### Example 6 : Therapeutic Effects Of Antibody In Combination With Cisplatin

The therapeutic effects of an anti-CD40 antibody (21.4.1) in preventing the growth of human breast tumors alone and In the presence of cisplatin was examined.

SCID-belge mice were injected SC with the breast tumor, BT 474. The antibody (1 mg/kg , IP) and/or cisplatin (2.5 mg/kg, IP) were administered as a single injection once tumors reached a size of 200 mm². Tumor growth was measured on Day 84 after challenge.

As shown in Figure 4, a single injection of 21.4.1 or cisplatin prevented tumor growth. However, the combination of both treatments lead to complete tumor regression in 7/8 animals.

21.4.1 prevents tumor growth when administered alone once tumors are established and causes tumor regression when administered in combination with cisplatin. This was also demonstrated using antibody 3.1.1 as is likely for 3.11H-A78T-V88A-V97A/3.1.1L-L4M-L83V as well.

### Example 7: Mullidose Pharmacokinetics of Antibody

in a multiple-dose study, 21.4.1 was administered intravenously to cynomolgus monkeys (2/sex/dose) at doses of 0.3, 1.0, and 10 mg/kg on Days 1, 3, 5,7, and 9 for 5 total doses. Blood was collected on Days 1 and 9 before dosing and 0.5, 6, and 24 hours after dosing and before dosing and 0.5 hour after dosing on Day 5 to measure serum drug concentrations. Systemic exposure to 21.4.1, as assessed by mean Cₘₐₓ and mean AUC₍₀₋₂₄₎, increased with increasing dose from 0.3 to 10 mg/kg on both Day 1 and Day 9 (Table 2). Similar exposures (mean Cₘₐₓ and mean AUC) were observed on Days 1 and 9 in the 0.3 and 1 mg/kg dose groups. In the 10 mg/kg dose group, the mean Cₘₐₓ and mean AUC₍₀₋₂₄₎ values increased 2.6- and 2.8-fold, respectively, from Day 1 to Day 9. Gender-related differences in exposure were not observed.

**Table 2**

| Mean (± SD) Pharmacokinetic Parameters of 21.4.1 in Cynomolgus Monkeys on Days 1 and 9 Following Every Other Day IV Administration | | | | |
|---|---|---|---|---|
| Dose^{a} | Day | Cₘₐₓ | Tₘₐₓ | AUC₍₀₋₂₄₎ |
| (mg/kg) | | (µg/mL) | (h) | (µg·h/mL) |
| 0.3 | 1 | 4.67 ± 1.71 | 1.9 ± 2.8 | 47.7 ± 15.4 |
| | 9 | 7.4 ± 2.9 | 0.5 ± 0.0 | 55.6 ± 47.1 |
| 1.0 | 1 | 28.7 ± 5.1 | 0.5 ± 0.0 | 387 ± 59 |
| | 9 | 12.3 ± 91 | 1.9 ± 2.8 | 219 ± 151 |
| 10 | 1 | 226 ± 29 | 1.9 ± 2.8 | 4130 ± 600 |
| | 9 | 577 ± 163 | 3.3 ± 3.2 | 11400 ± 2100 |

| | | | | |
|---|---|---|---|---|
| N = 2/sex/dose | | | | |

### Exemple 8: Antibody Formulation

CD40 antibody was concentrated to approximately 11.0 mg/mL ± 0.8 mg/mL using an ultrafiltration unit containing 30 kDa molecular weight cut-off cassettes. The concentrate was then diafiltered into 20 mM sodium acetate / 140 mM sodium chloride, pH 5.5 buffer. 2% polysorbate 80 solution was added to the concentrated diafiltered product to achieve a final concentration of 0.02% Polysorbate 80.

The invention also relates to the following aspects as defined in the following numbered paragraphs:
1. A method of treating cancer in a patient in need of such treatment comprising administering to said patient a CD40 agonist antibody or a fragment thereof, wherein said antibody is administered according to an intermittent dosing regimen of at least two cycles, each cycle comprising (a) a dosing period during which a therapeutically effective amount of said CD40 agonist antibody is administered to said patient and, thereafter, (b) a resting period
2. The method of paragraph 1 wherein the therapeutical effective amount produces a plasma concentration of said antibody of 0.01 µg/ml to 10 µg/ml for at least three hours and the resting period is for at least 1 week.
3. The method according to paragraph 1, wherein the therapeutically effective amount produces a plasma concentration of 0.03 µg/ml to 1.0 µg/ml.
4. The method according to paragraph 1, wherein the therapeutically effective amount maintains a plasma concentration of 0.1 µg/ml to 0.3 µg/ml.
5. The method according to paragraph 1, wherein the antibody is selected from the group consisting of an antibody having the amino acid sequence of antibody 3.1.1, 3.1.1H- A78T, 3.1.1 H-A78T-V88A-V97A, 3.1.1H-A78T-V88A-V97A/3. 1.1 L-L4M-L83V, 71.2.10.8.3, 15.1.1, 21.4.1, 21.2.1, 22.1.1, 22.1.1H-C109A, 23.5.1, 23.25.1, 23.28.1, 23.28 1H-D16E, 23.29.1, 24.2.1, 3.1.1 L-L4M-L83V and 23.28.1L-C92A.
6. The method according to paragraph 1, wherein the antibody comprises a CDR or a variable region of an antibody selected from the group consisting of an antibody designated 3.1.1, 3.1.1H-A78T, 3.1.1H-A78T-V88A-V97A, 3.1.1H-A78T-V88A-V97A/3.1.1L-L4M-L83V, 7.1.2, 10.8.3, 15.1,1, 21.4.1, 21.2.1, 22.1.1, 22.1.1H-C109A, 23.5 1, 23 25.1, 23.28.1, 23.28 1H-D16E, 23.29.1, 24. 2.1, 3.1. 1 L-L4M-L83V and 23.28.1L-C92A.
7. The method according to paragraph 1, wherein the antibody binds the same epitope as a CD40 antibody selected from the group consisting of an antibody designated 3.1.1, 3.1.1H-A78T, 3.1.1H-A78T-V88A-V97A, 3.1.1L-L4M-L83V, 31.1H-A78T-V88A-V97A/3.1.1L-L4M-L83V, 7.1.2, 10.8.3, 15.1.1, 21.4.1, 21.2.1, 22.1 1, 22.1.1H-C109A, 23.5.1, 23.25.1, 23.28.1, 23.28.1H-D16E, 23.29.1, 24..2.1, and 23.28.1 L-C92A.
8. The method according to paragraph 1, wherein the antibody competes with a CD40 antibody selected from the group consisting of an antibody designated 3.1.1, 3. 1.1H-A78T, 3.1.1H-A78T-V88A-V97A, 7.1.2, 10.8.3, 15.1.1, 21.4.1, 21.2.1, 22.1.1, 22.1.1H-C109A, 23.5.1, 23.25.1, 23.28.1, 23.28.1H-D16E, 23.29.1, 24.2.1, 3.1.1HA78T-V88A-V97A/3.1.1L-L4M-L83V and 23.28.1L-C92A.
9. The method according to paragraph 1, wherein the antibody has the amino acid sequence of an antibody selected from the group consisting of 21.4.1, 3.1.1, and 3.1.1 H-A78T-V88A-V97A/3.1.1L-L4M-L83V.
10. A method of treating a tumor in a patient in need of such treatment comprising administering to said patient a CD40 agonist antibody or a fragment thereof, wherein said antibody is administered according to an intermittent dosing regimen of at least two cycles, each cycle comprising (a) a dosing period of 1-5 days during which 0.03 to 3.0 mg/kg/day of the antibody is administered and, thereafter, (b) a resting period from 1 to 8 weeks.
11. The method according to paragraph 10, wherein 0.1 to 1.0 mg/kg/day or 0.1 to 0.3 mg/kg/day of the antibody is administered.
12. A method for treating tumor in a patient in need of such treatment comprising administering to said patient a combination of a therapeutically effective amount of a CD40 agonist antibody and a therapeutically effective amount of a DNA replication inhibitor.
13. A stable liquid pharmaceutical formulation suitable for parenteral administration comprising a CD40 agonist antibody at a pH of from 5.0-6. 0 and a pharmaceutical acceptable carrier, said formulation being stable for a period of at least three months.
14. The formulation of paragraph 13 having a concentration of said CD40 antibody of at least about 5 mg/ml.
15. The formulation of paragraph 13 comprising an anti-CD40 antibody, sodium acetate, sodium chloride, and polysorbate 80.
16. The formulation of paragraph 13 wherein said anti-CD40 antibody has the amino acid sequence of an antibody selected from the group consisting of 21.4.1, 3.1.1, and 3.1.1H- A78T-V88A-V97A/3.1.1L-L4M-L83V.
17. A method of treating a tumor in a patient in need of such treatment comprising administering to said patient a CD40 agonist antibody or a fragment thereof in a dosage of less than 1 mg/kg wherein the Cₘₐₓ serum concentration of said. antibody in said patient is less than 50 µg/ml.
18. The method of paragraph 17 wherein the dosage is between 0.1 to 0.3 mg/kg and wherein the Cₘₐₓ serum concentration of said antibody in said patient is between 0.5 and 10 µg/ml.
19. A method of enhancing immune response in a patient in need thereof comprising administering to said patient a CD40 agonist antibody or a fragment thereof, wherein said antibody is administered according to an intermittent dosing regimen of at least two cycles, each cycle comprising (a) a dosing period during which a therapeutical effective amount of said CD40 agonist antibody is administered to said patient and, thereafter, (b) a resting period.
20. Use of a CD40 agonist antibody, or a fragment thereof, for the manufacture of a medicament for treating cancer in a patient in need of such treatment by administering to said patient said CD40 agonist antibody or a fragment thereof, wherein said antibody is administered according to an intermittent dosing regimen of at least two cycles, each cycle comprising (a) a dosing period during which a therapeutical effective amount of said CD40 agonist antibody is administered to said patient and, thereafter, (b) a resting period.

## Claims

1. A liquid pharmaceutical formulation suitable for parenteral administration, comprising a CD40 agonist antibody and a pharmaceutically acceptable carrier, wherein the formulation is at pH 5.5, wherein the antibody consists of antibody 21.4.1, and wherein the pharmaceutically acceptable carrier comprises sodium acetate, sodium chloride, and polysorbate 80.

2. The formulation of claim 1, wherein the concentration of said CD40 antibody is at least 5mg/ml.

3. The formulation of claim 2, wherein the amount of sodium acetate is 20mM, the amount of sodium chloride is 140mM, and the amount of polysorbate 80 is 0,2mg/ml.

## Patentansprüche

1. Flüssige pharmazeutische Formulierung, geeignet für die parenterale Verabreichung, enthaltend einen CD40-Agonist-Antikörper und einen pharmazeutisch verträglichen Träger, wobei die Formulierung einen pH-Wert von 5,5 besitzt, worin der Antikörper aus dem Antikörper 21.4.1 besteht und worin der pharmazeutisch verträgliche Träger Natriumacetat, Natriumchlorid und Polysorbat 80 umfasst.

2. Formulierung gemäß Anspruch 1, wobei die Konzentration des besagten CD40-Antikörpers mindestens 5 mg/ml beträgt.

3. Formulierung gemäß Anspruch 2, wobei die Menge an Natriumacetat 20 mM beträgt, die Menge an Natriumchlorid 140 mM beträgt und die Menge an Polysorbat 80 0,2 mg/ml beträgt.

## Revendications

1. Formulation pharmaceutique liquide convenant à une administration parentérale, comprenant un anticorps agoniste de CD40 et un support pharmaceutiquement acceptable, la formulation étant à pH 5,5, l'anticorps consistant en l'anticorps 21.4.1, et le support pharmaceutiquement acceptable comprenant de l'acétate de sodium, du chlorure de sodium et du polysorbate 80.

2. Formulation selon la revendication 1, dans laquelle la concentration dudit anticorps CD40 est d'au moins 5 mg/ml.

3. Formulation selon la revendication 2, dans laquelle la quantité d'acétate de sodium est de 20 mM, la quantité de chlorure de sodium est de 140 mM et la quantité de polysorbate 80 est de 0,2 mg/ml.
